# EUROPEAN PATENT APPLICATION

(11) **EP 3 689 329 A1**
(43) Date of publication of application: **05.08.2020**
(21) Application number: 18861914.2
(22) Date of filing: 28.09.2018
(51) Int. Cl.: A61K 8/97, A23L 33/105, A61K 36/8888, A61K 36/31, A61K 36/27, A61K 36/258, A61Q 19/08

(54) **COMPOSITION COMPRISING PLANT-DERIVED EXTRACELLULAR VESICLES**

(30) Priority: 28.09.2017 KR 20170125906
(71) Applicant: Prostemics Co., Ltd., Seoul 06061 (KR)
(72) Inventor: JUNG, Soo Young, Seoul 02026 (KR); PARK, Eun Joo, Seoul 04805 (KR); CHOI, Eun Wook, Seoul 08098 (KR); LEE, Won Jong, Seoul 04732 (KR)
(74) Representative: Schnappauf, Georg
(86) International application number: PCT/KR2018/011508
(87) International publication number: WO 2019/066538

(57) **Abstract**

The present invention relates to various uses of a composition comprising plant-derived extracellular vesicles.

## Description

### Technical Field

The present invention relates to various uses of a composition comprising plant-derived extracellular vesicles.

### Background Art

Recently, with improved medical technology and public health benefits, a super-aged society is rapidly arriving. As a result, qualitative improvement in life is being pursued, and the desire to maintain youth is also increasing. Many studies are conducted in various fields to delay and prevent aging in physical appearance, in particular, aging which occurs first on the skin.

Wrinkles, on the other hand, are caused by aging on the skin, and aged skin is a totality of natural changes associated with aging processes. Skin aging is classified largely into two types: physiological aging that shows age-related changes in skin function, structure, or shape throughout the skin surface; and photo-aging caused by ultraviolet rays.

As skin aging progresses, remarkable changes in the dermis appear, and dermal atrophy that appears in people aged 70 or over is a typical aging phenomenon. Changes in the dermis occur due to changes in high molecular weight substances in the extracellular matrix, caused by a decrease in the number of fibroblasts and their synthesis ability. Specific examples of such changes may include separation of collagen bundles, decreased synthesis of mucopolysaccharides, decreased number and diameter of collagen and elastin, decomposition of collagen and elastin, expanded blood vessel, and the like. Generally, among various multiple factors, including the skin's moisture content, collagen content, and ability to exert an immune action to external environments, the greatest influence on formation of wrinkles is the expression level and activity of collagenase, a collagen-degrading enzyme that decreases collagen production and collagen content.

Human skin color is determined by the concentration and distribution of melanin in the skin. Melanin pigment produced by melanocytes of human skin is a phenolic polymer having a complex form of black pigment and protein, and plays an important role in blocking skin damage caused by ultraviolet rays. In melanin biosynthesis, the action of tyrosinase present in melanocytes is reported to be most important, and tyrosinase plays a key role in the skin darkening process by converting tyrosine, which is a type of amino acid, into DOPA and dopaquinone which are intermediate products in melanin polymer production.

On the other hand, hormonal imbalance is becoming more rampant due to environmental pollution, automobile exhaust gases, and the like. As a result, the incidence of hair loss is increasing, the age at which hair loss starts is also decreasing, and disharmony of skin immune system occurs, which causes various skin diseases such as atopy or psoriasis. In addition, despite their young age, the population suffering from obesity is growing rapidly as the diet has shifted toward instant food and meat.

Therefore, in-depth studies have been conducted to develop substances that can effectively solve various phenomena which are not only problematic on the aesthetic level, but also are becoming serious social problems, such as wrinkle formation, melasma, or freckles, caused by intrinsic aging and UV rays, obesity, immune imbalance, and hair loss.

### Technical Problem

An object of the present invention is to provide compositions comprising plant-derived extracellular vesicles, the compositions having various effects such as skin improvement, hair loss treatment, and wound treatment.

### Solution to Problem

The skin is composed of epidermis, dermis, and subcutaneous tissue. The epidermis, the outermost layer of skin, is composed of stratified squamous epithelium and acts as a protective barrier of the body against external environments. Starting from the outside, the epidermis is divided into stratum corneum, stratum lucidum, stratum granulosum, stratum spinosum, and stratum basale. The dermis, a layer between the epidermis and the subcutaneous tissue, is divided into papillary dermis and reticular dermis, and contains blood vessels, collagen, elastin fibers, pores, piloerector muscles, sebaceous glands, sweat glands, various sensory nerves, fibroblasts, macrophages, and the like which are not present in the epidermis. The dermis occupies the largest portion of the skin.

The dermis is composed mainly of collagen and elastin fibers which serve to support the skin. Therefore, in a case where problems occur in the dermis, wrinkles are formed and skin elasticity is lost, which causes skin aging to progress. Collagen is known to play the most important role in skin regeneration, skin moisture content, wound healing, wrinkle improvement, and the like, and is produced by fibroblasts. Collagen has an ability to contain a large amount of moisture, and thus serves to supply moisture to the dermis. As the skin ages, the collagen's ability to contain moisture decreases, and thus wrinkles increase. In addition, when a wound occurs, collagen also acts to heal the wound by filling the wounded area through its continuous production by fibroblasts.

In addition, in modern society, the living human body is gradually damaged due to a lot of stress, and hair loss caused thereby becomes a major social issue. Hair loss is caused by a variety of factors, including poor hair growth and lack of growth factors that promote growth of hair root cells.

Such hair loss deteriorates quality of life due to mental stress and causes a lot of inconvenience to interpersonal relations or social life. In social life, a personal image coming from physical appearance leaves a very impressive memory, and the physical appearance gives invisible energy to self-esteem or social life. From this point of view, there is a growing interest in the cause and treatment of hair loss.

It has been reported that when a substance is effective for hair loss, the substance is also effective for wound healing (see Korean Patent publication No. 10-2013-0075981). This is because signal transduction systems thereof are shared or influenced by each other. Indeed, according to an embodiment of the present invention, it can be said that in a case where a substance has a hair growth effect, the substance also has a wound healing effect.

The present inventors have discovered that extracellular vesicles derived from plants, in particular, Green dragon, Rapeseed, Hasuo (including Pleuropterus multiflorus and Cynanchum wilfordii), or wild ginseng adventitious root have excellent skin permeability; are excellent in effects such as wound healing, skin moisturization, skin whitening, wrinkle improvement, anti-aging, and the like when absorbed into the skin; and additionally have excellent hair growth and hair growth-promoting effects; and thus have arrived at the present invention.

According to an embodiment of the present invention, there is provided a cosmetic composition for skin improvement, comprising plant-derived extracellular vesicles as an active ingredient. More preferably, the extracellular vesicles may be contained at a concentration of 5 to 10 µg/ml.

According to another embodiment of the present invention, there is provided a food composition for skin improvement, comprising plant-derived extracellular vesicles as an active ingredient. More preferably, the extracellular vesicles may be contained at a concentration of 5 to 10 µg/ml.

According to yet another embodiment of the present invention, there is provided a pharmaceutical composition for wound healing, comprising plant-derived extracellular vesicles as an active ingredient. More preferably, the extracellular vesicles may be contained at a concentration of 5 to 10 µg/ml.

According to still yet another embodiment of the present invention, there is provided a pharmaceutical composition for hair loss prevention or treatment, comprising plant-derived extracellular vesicles as an active ingredient. More preferably, the extracellular vesicles may be contained at a concentration of 5 to 10 µg/ml.

According to still yet another embodiment of the present invention, there is provided a cosmetic composition for hair loss improvement, comprising plant-derived extracellular vesicles as an active ingredient. More preferably, the extracellular vesicles may be contained at a concentration of 5 to 10 µg/ml.

According to still yet another embodiment of the present invention, there is provided a food composition for hair loss improvement, comprising plant-derived extracellular vesicles as an active ingredient. More preferably, the extracellular vesicles may be contained at a concentration of 5 to 10 µg/ml.

As used herein, the term "extracellular vesicle" refers to a membranous small sac secreted from various cells and is also defined as nanovesicle. The extracellular vesicles have a diameter of approximately 30 to 1,000 nm, and preferably have an average diameter of 100 to 300 nm. The extracellular vesicles mean sacs that are released into the extracellular environment by fusion between multivesicular bodies and plasma membrane.

As used herein, the term "plant" is understood to have the meaning of including not only mature plants, but also plant cells, plant tissues, plant's seeds, and the like which can develop into mature plants.

The types of plants that can be used in the present invention are not particularly limited. For example, a plant selected from the group consisting of Green dragon, Rapeseed, Hasuo and tissue-cultured wild ginseng root may be used. However, the types of plants that can be used in the present invention are not limited to the above-mentioned types, and any material may be used without limitation as long as the material belongs to plants.

As used herein, the "wild ginseng" is an example of a plant containing a large number of functional ingredients, and is ginseng naturally growing in the mountains which belongs to Panax ginseng C.A. Meyer(Araliaceae). The wild ginseng, a semi-shade plant, also belongs to a phanerogam, in which when buds come out, a stalk for bearing flowers comes with leaves and stems, and also to an angiosperm. The wild ginseng was recorded as "god's grass" in Donguibogam, an encyclopedia of traditional Korean medicine, and is a rare plant having been treated as a mysterious elixir from old times. The wild ginseng has been used as a therapeutic agent for various diseases and as a medicine to aid virility. The wild ginseng is classified into four types: Cheonjong, Jijong, Injong, and cultivated wild ginseng. Specifically, Cheonjong (natural wild ginseng) is a product of a seed fallen from wild ginseng that grew naturally in the mountains; Jijong is a product of a wild ginseng's seed that has been ingested by wild animals or birds and excreted in the mountains; Injong is a product of a wild ginseng's seed sown by a human; and the cultivated wild ginseng (cultivated wild ginseng) is a product obtained by harvesting a wild ginseng's seed, seeding it deep in the mountains, and performing artificial cultivation in a wild state. In general, the wild ginseng is a very rare plant that is difficult to cultivate from the viewpoints that the wild ginseng does not sprout well even when sown, and that even if the wild ginseng grows, it has often rotten away in a few years.

In the present invention, as the wild ginseng, wild ginseng adventitious root may be used. Here, the wild ginseng adventitious root may be obtained by plant tissue culture methods known to those skilled in the art, for example, the methods described in Korean Patent Nos. 0353636 and 0392513, and the like. The production methods thereof are not particularly limited.

As used herein, the term "Green dragon", having the scientific name of "Pinellia tripartita", refers to a plant belonging to the Araceae family. Green dragon is a perennial plant and has a round corm in the ground. The fruit of Green dragon is an oval juicy berry with seeds in it and ripens green.

As used herein, the tenn "Rapeseed", having the scientific name of "Brassica napus", refers to a biennial, dicotyledonous plant belonging to the cruciferous family and the Papaverales order. Flowers thereof bloom in racemose form around April and run on the ends of branches. The fruit thereof is in a columnar shape with a long beak at the end and has a seam in the middle, in which when ripened, the seam splits and 20 dark brown seeds come out. Propagation of Rapeseed is made by seeds. Rapeseed is distributed in Japan, China, and Korea. In Korea, it can be found throughout the country and is cultivated mainly in the south.

As used herein, the term "Hasuo" refers to white one in two types of Hasuo, that is, white one (Cynanchum wilfordii) and red one (Pleuropterus multiflorus). The white Hasuo belongs to Cynanchum wilfordii, Hemsley (Asclepiadaceae) and is known to be used for weakness after disease, anemia, premature hair graying, nervous breakdown, chronic stroke with limbs dysfunction, and the like due to its efficacy of invigorating vital energy and nourishing the blood.

In addition, in the present invention, the pharmaceutical composition may further contain an appropriate carrier, excipient, or diluent according to conventional methods. As the carrier, excipient, and diluent which may be contained in the pharmaceutical composition of the present invention, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, methyl hydroxybenzoate, propyl hydroxybenzoate, talc, magnesium stearate, mineral oil, and the like may be used. However, the carrier, excipient, and diluent are not limited thereto.

In addition, the pharmaceutical composition according to the present invention may be formulated in the form of oral preparations such as powders, granules, tablets, capsules, suspensions, emulsions, syrups, aerosols and the like, preparations for external use, suppositories, and sterile injectable solutions, respectively, according to conventional methods, and used. Specifically, in a case of being made into formulations, the formulations may be prepared by using commonly used diluents or excipients such as fillers, extenders, binders, wetting agents, disintegrants, and surfactants. Solid formulations for oral administration include tablets, pills, powders, granules, capsules, and the like. Such solid formulations may be prepared by mixing the above pharmaceutical composition of the present invention with at least one excipient, for example, starch, calcium carbonate, sucrose, lactose, gelatin, and the like. In addition, besides simple excipients, lubricants such as magnesium stearate and talc may also be used. Liquid formulations for oral administration include suspensions, solutions, emulsions, syrups, and the like. The liquid formulations can contain various excipients such as wetting agents, sweetening agents, fragrances, and preservatives, in addition to water and liquid paraffin which are commonly used simple diluents. Formulations for parenteral administration include sterile aqueous solutions, non-aqueous solutions, suspensions, emulsions, freeze-dried formulations, and suppositories. For the non-aqueous solutions and the suspensions, propylene glycol, polyethylene glycol, vegetable oil such as olive oil, injectable ester such as ethyl oleate, and the like may be used. As bases for the suppositories, Witepsol, macrogol, Tween 61, cacao fat, laurin fat, glycerogelatin, and the like may be used.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred. As used herein, the term "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

In the present invention, the food composition may be prepared in the form of various foods, for example, beverages, gums, tea, vitamin complexes, powders, granules, tablets, capsules, confections, rice cakes, bread, and the like. The food composition of the present invention is composed of a plant extract having little toxicity and side effects, and thus can be used without worries in a case of being ingested for a long time for preventive purposes.

In a case where the plant-derived extracellular vesicles or a filtrate containing the same, each of which is to be contained as an active ingredient in the present invention, is contained in the food composition, the amount thereof to be added may be 0.1 % to 50% of a total weight of the food composition.

Here, in a case where the food composition is prepared in the form of a beverage, there is no particular limitation except that the beverage contains the food composition at an indicated proportion, and the beverage may contain various flavoring agents or natural carbohydrates, or the like as additional ingredients similarly to conventional beverages. That is, examples of the natural carbohydrates may include monosaccharides such as glucose, disaccharides such as fructose, polysaccharides such as sucrose, conventional sugars such as dextrin and cyclodextrin, and sugar alcohol such as xylitol, sorbitol, and erythritol. Examples of the flavoring agents may include natural flavoring agents (thaumatin, stevia extracts (such as rebaudioside A), glycyrrhizin, and the like) and synthetic flavoring agents (saccharin, aspartame, and the like).

In addition, the food composition of the present invention may contain various nutrients, vitamins, minerals (electrolytes), flavorings such as synthetic flavorings and natural flavorings, colorants, pectic acid and salts thereof, alginic acid and salts thereof, organic acids, protective colloidal thickeners, pH adjusting agents, stabilizers, preservatives, glycerin, alcohol, carbonizing agents used in carbonated beverages, and the like.

These ingredients may be used individually or in combination. The proportion of such additives is not so important, and is generally selected from the range of 0.1 to about 50 parts by weight per 100 parts by weight of the food composition of the present invention.

In addition, in the present invention, the cosmetic composition may be prepared in the form of skin softeners, nourishing lotions, nourishing essences, massage creams, cosmetic bath water additives, body lotions, body milks, bath oil, baby oil, baby powders, shower gels, shower creams, sun screen lotions, sun screen creams, suntan creams, skin lotions, skin creams, UV blocking cosmetics, cleansing milks, hair removing agents (for cosmetic purposes), face and body lotions, face and body creams, skin whitening creams, hand lotions, hair lotions, cosmetic creams, Jasmine oil, bath soaps, liquid soaps, cosmetic soaps, shampoos, hand cleaners, medicinal soaps (for non-medical purposes), cream soaps, facial washes, body cleansers, scalp cleansers, hair rinses, toilet soaps, tooth whitening gels, toothpastes, and the like. To this end, the composition of the present invention may further contain either a solvent which is conventionally used for the preparation of cosmetic compositions, or a suitable carrier, excipient, or diluent.

The type of solvent that may further be added to the cosmetic composition of the present invention is not particularly limited, and examples of the solvent may include water, saline, DMSO, or a combination thereof. In addition, examples of the carrier, excipient, or diluent include, but are not limited to, purified water, oil, wax, fatty acids, fatty acid alcohol, fatty acid esters, surfactants, humectants, thickeners, antioxidants, viscosity stabilizers, chelating agents, buffers, lower alcohol, and the like. In addition, the cosmetic composition of the present invention may, if necessary, contain whitening agents, moisturizing agents, vitamins, UV blocking agents, fragrances, dyes, antibiotics, antibacterial agents, and antifungal agents.

Examples of the oil may include hydrogenated vegetable oil, castor oil, cottonseed oil, olive oil, palm kernel oil, jojoba oil, and avocado oil, and examples of the wax may include beeswax, spermaceti, carnauba wax, candelilla wax, montan wax, ceresin wax, liquid paraffin, and lanolin.

Examples of the fatty acids may include stearic acid, linoleic acid, linolenic acid, and oleic acid; examples of the fatty acid alcohol may include cetyl alcohol, octyl dodecanol, oleyl alcohol, panthenol, lanolin alcohol, stearyl alcohol, and hexadecanol; and examples of the fatty acid esters may include isopropyl myristate, isopropyl palmitate, and butyl stearate. Examples of the surfactants may include cationic surfactants, anionic surfactants, and nonionic surfactants, which are known in the art. Among these, if possible, surfactants derived from natural products are preferred.

In addition, the cosmetic composition of the present invention may contain humectants, thickeners, antioxidants, and the like, which are widely known in the cosmetic field, and the types and amounts thereof are as known in the art.

In an embodiment according to the present invention, there is provided a cosmetic composition for hair-loss prevention or hair growth, comprising plant-derived extracellular vesicles as an active ingredient. In the cosmetic composition of the embodiment, the plant is at least one selected from the group consisting of Green dragon, Rapeseed, Hasuo and wild ginseng adventitious root. In addition, in an embodiment according to the present invention, there is provided a food composition for hair-loss prevention or hair growth, comprising plant-derived extracellular vesicles as an active ingredient. In the food composition of the embodiment, the plant is at least one selected from the group consisting of Green dragon, Rapeseed, Hasuo and wild ginseng adventitious root. In addition, in an embodiment according to the present invention, there is provided a pharmaceutical composition for hair-loss prevention or hair growth, comprising plant-derived extracellular vesicles as an active ingredient. In the pharmaceutical composition of the embodiment, the plant is at least one selected from the group consisting of Green dragon, Rapeseed, Hasuo and wild ginseng adventitious root.

In an embodiment according to the present invention, there is provided a cosmetic composition for wound healing or improvement, comprising plant-derived extracellular vesicles as an active ingredient. In the cosmetic composition of the embodiment, the plant is at least one selected from the group consisting of Green dragon, Rapeseed, Hasuo and wild ginseng adventitious root. In addition, in an embodiment according to the present invention, there is provided a food composition for wound healing or improvement, comprising plant-derived extracellular vesicles as an active ingredient. In the food composition of the embodiment, the plant is at least one selected from the group consisting of Green dragon, Rapeseed, Hasuo and wild ginseng adventitious root. In addition, in an embodiment according to the present invention, there is provided a pharmaceutical composition for wound healing, comprising plant-derived extracellular vesicles as an active ingredient. In the pharmaceutical composition of the embodiment, the plant is at least one selected from the group consisting of Green dragon, Rapeseed, Hasuo and wild ginseng adventitious root.

In an embodiment according to the present invention, there is provided a method for growing hair or preventing hair loss, comprising a step of administering, to a subject in need thereof, an effective amount of plant-derived extracellular vesicles as an active ingredient. In the method of the embodiment, the plant is at least one selected from the group consisting of Green dragon, Rapeseed, Hasuo and wild ginseng adventitious root.

In an embodiment according to the present invention, there is provided a composition for hair-loss prevention or hair growth, comprising plant-derived extracellular vesicles as an active ingredient. In the composition of the embodiment, the plant is at least one selected from the group consisting of Green dragon, Rapeseed, Hasuo and wild ginseng adventitious root.

### Advantageous Effects of Invention

The plant-derived extracellular vesicles provided in the present invention have excellent skin permeability; have excellent skin improvement effects such as skin moisturization, skin whitening, wrinkle improvement, and anti-aging, when absorbed into the skin; and are safely used without causing irritation or side effects on the skin.

In addition, the plant-derived extracellular vesicles provided in the present invention promote cell proliferation at a wounded area, and result in an increased expression level of growth factors and the like which have an excellent wound healing effect, thereby exhibiting excellent wound healing activity or wound healing promotion activity.

In addition, the plant-derived extracellular vesicles provided in the present invention have excellent hair growth and hair growth-promoting effects, and thus exhibit an excellent effect of preventing and treating hair loss; and are safe from the viewpoint that there are no side effects in a case of being ingested or applied to the skin.

### Brief Description of Drawings

FIG. 1 illustrates results analyzed by Western blotting for the amount of hair growth-related PCNA, according to an embodiment of the present invention.
FIG. 2 illustrates results for the density value level of hair growth-related PCNA, according to an embodiment of the present invention.
FIG. 3 illustrates results for the relative mRNA level with respect to the expression level of hair growth-related genes, according to an embodiment of the present invention.
FIG. 4 illustrates results obtained by measuring the length of hair follicles isolated from the human scalp tissue, according to an embodiment of the present invention.

### Detailed Description of Invention

Hereinafter, the present invention will be described in detail by way of the following examples. However, the following examples are merely given to illustrate the present invention, and the scope of the present invention is not limited to the following examples.

### Example 1. Isolation of microvesicles

Tissue culture or extraction juice of each of Green dragon, Rapeseed, Cynanchum wilfordii, and wild ginseng adventitious root was centrifuged at 4°C for 10 minutes. The supernatant was taken and centrifuged at 1,200 g for 20 minutes at 4°C. Then, the supernatant was taken and centrifuged at 10,000 g for 30 minutes at 4°C. The supernatant was taken and centrifuged using an ultracentrifuge at 110,000 g for 1 hour and 10 minutes at 4°C. Then, the supernatant was removed, and the pellet was suspended in PBS to isolate microvesicles.

### Example 2. Identification of efficacy of isolated microvesicles

### Identification of expression of hair growth-related protein

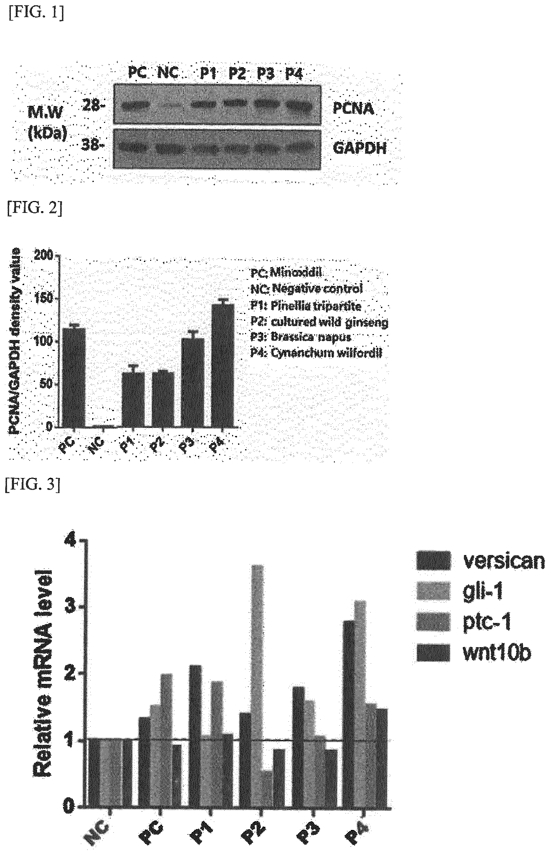

In order to identify an effect of the microvesicles of Example 1 on the protein level of a hair growth-related gene, treatment with the microvesicle samples was performed, and then the amounts of PCNA were compared and identified through Western blotting (see FIGS. 1 and 2). As a result, it was found that all microvesicles derived from Green dragon, soybean, Rapeseed, Cynanchum wilfordii, and cultured wild ginseng root result in a protein expression level equal to or higher than the positive control, minoxidil. In particular, the Cynanchum wilfordii-derived microvesicles resulted in about 20% higher protein expression level.

### Identification of expression of hair growth-related genes

In order to further verify the microvesicles' hair growth-promoting effect, expression of hair growth-related genes was checked (see FIG. 3). As a result, although there was a difference in tendency of expression for the respective genes, it was identified that microvesicles derived from Green dragon, soybean, Rapeseed, Cynanchum wilfordii, and cultured wild ginseng root result in increased gene expression. In particular, the Cynanchum wilfordii-derived microvesicles resulted in remarkably higher gene expression than the positive control, minoxidil, for the checked 4 gene types.

### Evaluation of effectiveness using human hair follicle tissue

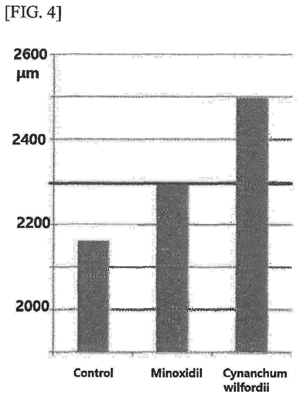

As an effectiveness evaluation method that can replace an animal test, effectiveness evaluation was conducted using human hair follicle tissue. Hair follicles were isolated from human scalp tissue to be discarded due to accident and surgery in the hospital and treated for 5 days with the positive control, minoxidil, and the sample microvesicles. Then, the lengths thereof were measured to evaluate the microvesicles' effectiveness on growth of hair follicles in terms of length (see FIG. 4). It was found that as compared with the positive control, the Cynanchum wilfordii-derived microvesicles result in a remarkable growth of hair follicles in terms of length.

The scope of the present invention is not limited to the examples as described above, and is defined by the appended claims. It will be obvious to those skilled in the art that various modifications and changes can be made without departing from the technical spirit of the present invention.

### Industrial Applicability

The plant-derived extracellular vesicles provided in the present invention have excellent skin permeability; have excellent skin improvement effects such as skin moisturization, skin whitening, wrinkle improvement, and anti-aging, when absorbed into the skin; and promote cell proliferation at a wounded area and result in an increased expression level of growth factors and the like which have an excellent wound healing effect, thereby exhibiting excellent wound healing activity or wound healing promotion activity. Accordingly, this active ingredient can be used in the form of a pharmaceutical composition, a cosmetic composition, and a food composition.

## Claims

1. A cosmetic composition for hair-loss prevention or hair growth comprising plant-derived extracellular vesicles as an active ingredient.

2. The cosmetic composition according to claim 1, wherein the plant is at least one selected from the group consisting of Green dragon, Rapeseed, Cynanchum wilfordii and wild ginseng adventitious root.

3. A food composition for hair-loss prevention or hair growth comprising plant-derived extracellular vesicles as an active ingredient.

4. The food composition according to claim 3, wherein the plant is at least one selected from the group consisting of Green dragon, Rapeseed, Cynanchum wilfordii and wild ginseng adventitious root.

5. A pharmaceutical composition for hair-loss prevention or hair growth comprising plant-derived extracellular vesicles as an active ingredient.

6. The pharmaceutical composition according to claim 5, wherein the plant is at least one selected from the group consisting of Green dragon, Rapeseed, Cynanchum wilfordii and wild ginseng adventitious root.

7. A cosmetic composition for wound healing or improvement comprising plant-derived extracellular vesicles as an active ingredient.

8. The cosmetic composition according to claim 7, wherein the plant is at least one selected from the group consisting of Green dragon, Rapeseed, Cynanchum wilfordii and wild ginseng adventitious root.

9. A food composition for wound healing or improvement comprising plant-derived extracellular vesicles as an active ingredient.

10. The food composition according to claim 9, wherein the plant is at least one selected from the group consisting of Green dragon, Rapeseed. Cynanchum wilfordii and wild ginseng adventitious root.

11. A pharmaceutical composition for wound healing comprising plant-derived extracellular vesicles as an active ingredient.

12. The pharmaceutical composition according to claim 11, wherein the plant is at least one selected from the group consisting of Green dragon, Rapeseed, Cynanchum wilfordii and wild ginseng adventitious root.

13. A method for growing hair or preventing hair loss comprising administering an effective amount of plant-derived extracellular vesicles as an active ingredient to a subject in need thereof.

14. The method according to claim 13, wherein the plant is at least one selected from the group consisting of Green dragon, Rapeseed, Cynanchum wilfordii and wild ginseng adventitious root.

15. A composition for hair-loss prevention or hair growth comprising plant-derived extracellular vesicles as an active ingredient.

16. The composition according to claim 15, wherein the plant is at least one selected from the group consisting of Green dragon, Rapeseed, Cynanchum wilfordii and wild ginseng adventitious root.
